(19)

Europäisches
Patentamt
European
Patent Office
Office européen
des brevets

(11) **EP 3 875 041 A1**

(12) **EUROPEAN PATENT APPLICATION**
published in accordance with Art. 153(4) EPC

(43) Date of publication:
 **08.09.2021 Bulletin 2021/36**

(21) Application number: **19879967.8**

(22) Date of filing: **01.11.2019**

(51) Int Cl.:
 **A61B 17/072** *(2006.01)*

(86) International application number:
 **PCT/CN2019/114934**

(87) International publication number:
 **WO 2020/088627 (07.05.2020 Gazette 2020/19)**

(84) Designated Contracting States:
 **AL AT BE BG CH CY CZ DE DK EE ES FI FR GB GR HR HU IE IS IT LI LT LU LV MC MK MT NL NO PL PT RO RS SE SI SK SM TR**
 Designated Extension States:
 **BA ME**
 Designated Validation States:
 **KH MA MD TN**

(30) Priority: **02.11.2018 CN 201811302987**

(71) Applicant: **Ezisurg Medical Co., Ltd.**
 **Shanghai 200000 (CN)**

(72) Inventors:
 • **TANG, Chuangang**
  **Shanghai 201318 (CN)**

 • **BAO, Minghui**
  **Shanghai 201318 (CN)**
 • **LIAO, Menghui**
  **Shanghai 201318 (CN)**
 • **YANG, Jun**
  **Shanghai 201318 (CN)**
 • **NIE, Honglin**
  **Shanghai 201318 (CN)**

(74) Representative: **Mansfield, Peter Turquand**
 **Mathys & Squire**
 **The Shard**
 **32 London Bridge Street**
 **London SE1 9SG (GB)**

(54) **SURGICAL INSTRUMENT HAVING BENDABLE ACTUATOR**

(57) The present application relates to a surgical instrument with a pivotable effector, including an effector at a distal end, a controller at a proximal end, and an extension tube between the effector and the controller, wherein the extension tube is movably connected to a distal end of the controller; a distal end of the extension tube is pivotally connected to a proximal end of the effector; the extension tube forms a connection channel for transferring an action of the controller to the effector; the controller includes a articulation control mechanism capable of moving in at least one direction; the extension tube includes a first flexible component, and moves in at least one direction through the articulation control mechanism, and the first flexible component provides a rotation torque for the effector, and drives the effector to rotate towards at least one side relative to the distal end of the extension tube. The articulation control mechanism provided by the present application has a simple solution, few components, and a low cost, is simple to manufacture, and realizes flexible articulation of the effector.

FIG. 2

EP 3 875 041 A1

## Description

## TECHNICAL FIELD

The present application relates to a medical instrument, and more particularly to a surgical instrument with a pivotable effector.

## BACKGROUND

A surgical stapler is a surgical instrument, and its operating principle is to clamp tissues by closing two corresponding effectors (which generally includes an anvil assembly and a staple cartridge assembly), and then pushing out metal suturing staples from the staple cartridge of the stapler to suture the tissues together. In some staplers, a cutter is also mounted, and is used to incise the sutured tissues.

With the progress of technology, conventional surgical methods are gradually turning to endoscopic operation. Endoscopic operation refers to: firstly, making several small incisions of 5-12 mm diameters at different positions in the abdomen or chest; then, a camera lens and various special surgical instruments are inserted through the small incisions; then, images of various organs in the abdominal cavity taken by the camera inserted into the abdominal cavity are transmitted to a television screen; and finally, a surgeon observes the images, and uses various surgical instruments to complete the operation, wherein the endoscopic stapler plays the most important role during operation.

However, endoscopic operation also has limitations. Due to the limitation of space in the abdominal cavity or the thoracic cavity, the conventional linear endoscopic stapler cannot effectively reach a surgical site to clamp, transversely cut, and staple the tissues under some extreme situations. Therefore, an endoscopic stapler with a pivotable stapler effector is produced.

The EndoGIA Universal cutting stapler and staple cartridge made by American Tyco Healthcare Company (later renamed as Covidien) are representative products to realize the above functions. The product in the US patent application No. US2010/0237131A1 adopts the structure of hinge + eccentric rigid link hinge to obtain the deflection of the effector. Limited by the structural design principle, the maximum rotating angle of the staple cartridge effector is only 45°. In a situation where an effector with a large rotating angle is required to complete an operation, an effector with a small rotating angle will be unable to remove the item of concern at some sites. For example, in low proctectomy, since the space of the pelvic cavity limits the angle of the instrument entering the pelvic cavity, although the staple cartridge effector can pivot, the effector still cannot suture perpendicularly and cut off the rectum in some operations, such that for a patient whose tumor position is too low, the instrument cannot be used for surgical treatment.

Our company provides a surgical instrument with a pivotable effector in the earlier application No. CN201510672007.2, and provides a surgical instrument and articulation control mechanism thereof in the earlier application No. CN201510026576.X. The two patents can realize 60° articulation of the staple cartridge effector. However, the staple cartridge effector and the manual articulation control mechanism belong to different products; when in use, a surgeon needs to assemble the two together to realize articulation control, thereby having a certain requirement for assembly accuracy.

Therefore, the present invention aims to design a surgical instrument capable of manually controlling articulation without the need for a surgeon to assemble.

## SUMMARY

According to the present application there is provided a surgical instrument with a pivotable effector, comprising an effector at a distal end, a controller at a proximal end, and an extension tube between the effector and the controller, wherein the extension tube is movably connected to a distal end of the controller; a distal end of the extension tube is pivotally connected to a proximal end of the effector; the extension tube forms a connection channel for transferring an action of the controller to the effector; the controller comprises an articulation control mechanism capable of moving in at least one direction; the extension tube comprises a first flexible component, and moves in at least one direction through the articulation control mechanism, and the first flexible component provides a rotation torque for the effector, and drives the effector to rotate towards at least one side relative to the distal end of the extension tube.

Preferably, the distal end of the extension tube is pivotally connected to the proximal end of the effector via a hinge; the first flexible component does not pass through the centre of the hinge, such that the first flexible component takes the hinge as a rotation axis, provides a rotation torque for the effector, and drives the effector to rotate to at least one side relative to the distal end of the extension tube.

Further, the effector rotates in a plane perpendicular to the hinge rotation axis.

According to one aspect, the articulation control mechanism includes an operating component, a articulation control gear, and a first articulation control rack; the operating component is connected to the articulation control gear, and drives the articulation control gear to rotate; the articulation control gear is engaged with the first articulation control rack; the rotation of the articulation control gear drives the first articulation control rack to move forward or backward; and the first articulation control rack is fixedly connected to a proximal end of the first flexible component.

Further, the extension tube further includes a second flexible component; a distal end of the first flexible component and a distal end of the second flexible component are respectively connected on the two sides of the effector along a central shaft; when the articulation control mechanism moves in a first direction, the articulation control mechanism drives the first flexible component to move towards a proximal end thereof, drives the second flexible component to move towards a distal end thereof, and then drives the effector to rotate to a first side relative to the distal end of the extension tube; and when the articulation control mechanism moves in a second direction, the articulation control mechanism drives the first flexible component to move towards the distal end thereof, drives the second flexible component to move towards the proximal end thereof, and then drives the effector to rotate to a second side relative to the distal end of the extension tube.

Further, the first flexible component and the second flexible component are segmented flexible components.

Further, the first flexible component and the second flexible component are a bendable pull beam or a pull cable.

Further, the articulation control mechanism further includes a second articulation control rack; the articulation control gear is engaged with the second articulation control rack; the rotation of the articulation control gear drives the second articulation control rack to move forward or backward; and the second articulation control rack is fixedly connected to a proximal end of the second flexible component.

Further, the operating component is a rotation knob. An operating region of the rotation knob is located outside the extension tube.

Further, the extension tube includes an outer sleeve tube, an upper inner tube, and a lower inner tube; the upper inner tube, the lower inner tube, and the outer sleeve tube are coaxially arranged.

Further, the first flexible component is a left bendable pull beam; the second flexible component is a right bendable pull beam; the first articulation control rack is a left articulation control rack; the second articulation control rack is a right articulation control rack; the left articulation control rack and the right articulation control rack are respectively disposed on the two sides of the lower inner tube; the left bendable pull beam and the right bendable pull beam respectively penetrate through a connecting assembly, and are connected to left and right sides of the effector along the central shaft; an upper side of the articulation control gear is connected to the rotation knob, such that the rotation of the rotation knob drives the articulation control gear to rotate, then drives the left articulation control rack and the right articulation control rack to move forward or backward, and correspondingly drives the left bendable pull beam to move forward or backward; and the right articulation control rack drives the right bendable pull beam to move forward or backward.

Further, a rotating head upper-cover and a rotating lower-cover which are connected to each other are disposed at a proximal end of the extension tube; the articulation control mechanism is located between the rotating head upper-cover and the rotating lower-cover; and the rotation knob is rotatably connected to the rotating head upper-cover via a rotation knob pin.

The rotation angle of the effector to at least one side relative to the distal end of the extension tube is any value between 0° and 180°.

A rotation angle $\alpha$ of the rotation knob basically can be losslessly fed back to an articulation angle $\beta$ of the effector, thereby having a high transmission efficiency; by adjusting the rotation angle $\alpha$ of the rotation knob 14, the articulation angle $\beta$ of the effector can be controlled, wherein $\alpha$ can be any value between 0° and 180°, for example, 0°, 30°, 45°, 60°, 90°, 120°, 135°, 180° and the like; and $\beta$ can be any value between 0° and 180°, for example, 0°, 30°, 45°, 60°, 90°, 120°, 135°, 180° and the like.

In a specific embodiment, the angle relationship between $\alpha$ and $\beta$ satisfies the following formula:

$$\beta = 0.9\,\alpha - 2.1$$

Further, the bendable pull beam is detachable, for example, one embodiment achieves detachment by means of a segmented bendable pull beam, that is, the left bendable pull beam and the right bendable pull beam are divided into two segments or multiple segments.

In another embodiment, two or more bendable pull beams can be placed in an overlapping manner.

Preferably, the surgical instrument is applied to an endoscopic stapler.

Further, the flexible components are a bendable pull beam or a wire rope.

The surgical instrument of the present application adopts the flexible components to pivot the effector relative to the distal end of the extension tube, such that the effector can pivot through a larger angle, thereby being applicable to medical treatment under more complicated clinical conditions, for example, the surgical instrument can more simply implement an operation in a limited space (such as a thoracic cavity or pelvic cavity). Therefore, the present application solves the problem that other surgical instruments cannot solve. The effector of the present application can pivot through a large angle; and the surgical instrument can complete the functions of excising an item of concern, tissue cutting and suturing at difficult locations, thereby benefiting more patients. For example, for a rectal cancer patient, the effector of the instrument of the present application still can rotate, at the lowermost location of a rectum, to an angle perpendicular to the rectum, such that a low rectal cancer patient can also accept a minimally invasive operation. Furthermore, the present application has few transmission parts; in one aspect, the manufacturing cost is low, and in another aspect, the tolerance accumulation of a transmission system is small; the present application enables detachment by configuring a segmented bendable pull beam, thereby facilitating use.

The surgical instrument of the present application not only can be used in a surgical operation, but also can be used during treatment and diagnosis. In addition, the present application not only can be applied to an endoscopic stapler, but also can be applied to products such as a closer, an electrotome and the like.

## BRIEF DESCRIPTION OF THE DRAWINGS

In order to more clearly describe the technical solution of the present application, the accompanying drawings required for describing the embodiments or the prior art are briefly introduced hereinafter. Obviously, the accompanying drawings described below are merely some embodiments of the present application, and a person skilled in the art may obtain other drawings according to the accompanying drawings without involving an inventive effort.

FIG. 1 is a design diagram of the surgical instrument according to the present application;

FIG. 2 is an exploded schematic diagram of the elliptic region in FIG. 1; and

FIG. 3 is a schematic diagram of the effector in a bending state according to the present application.

In the drawings: 1: effector; 2: connector assembly; 3: left bendable pull beam; 4, 5: cutter bar protection plate; 6: connecting sheet; 7: outer sleeve tube; 8: upper inner tube; 9: cutter frame assembly; 10: center rod assembly; 11: right bendable pull beam; 12: lower inner tube; 13: rotation knob pin; 14: rotation knob; 15: rotating head upper-cover; 16: articulation control gear; 17: right articulation control rack; 18: left articulation control rack; 19: rotating head lower-cover; 20: extension tube; 30: controller; 31: movable handle; and 32: stationary handle.

## DETAILED DESCRIPTION OF THE EMBODIMENTS

The technical solutions in the embodiments of the present application are clearly and completely described below with reference to the accompanying drawings of the description. It will be apparent that the described embodiments are merely some rather than all of the embodiments of the present application. On the basis of the embodiments in the present application, all the other embodiments obtained by a person skilled in the art without involving an inventive effort are all included in the protection scope of the present application.

The term "exemplary" is used herein to represent "serving as an example, an instance or illustration". Any implementation described as "exemplary" herein is not necessarily asserted to be superior to or better than other implementations. Furthermore, "proximal end", "proximal side", and "back" herein refer to the end close to an operator; and "distal end", "distal side", and "front" refer to the end distal from the operator.

For simplicity, the instrument of the present application is described by taking a stapler as an example. A person skilled in the art can understood that the surgical instrument with a pivotable effector of the present application is not limited to an endoscopic stapler. The technology of the present application can also be applied to other products such as a closer, an electrotome and the like.

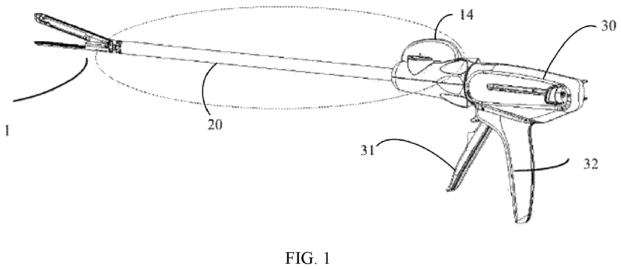

FIG. 1 is a design diagram of the surgical instrument according to the present application. Preferably, the surgical instrument is a stapler. The stapler comprises an effector 1 at a distal end, a controller 30 at a proximal end, and an extension tube 20 between the effector 1 and the controller 30, wherein the extension tube 20 is movably connected to a distal end of the controller 30; a distal end of the extension tube 20 is pivotally connected to a proximal end of the effector 1 via, preferably, one or more hinges; the extension tube 20 forms a connection channel for transferring an action of the controller 30 to the effector 1; the controller 1 is controlled to rotate by operating the controller 30; preferably, the effector rotates a certain angle in a plane perpendicular to a rotary shaft of the hinge.

The surgical instrument of the present application can adopt an integral design. That is, the extension tube 20, as a whole, is respectively connected to the distal end of the controller 30 and the proximal end of the effector 1. The length of the extension tube 20 can be different according to different indications and specific situations of a patient.

The surgical instrument of the present application can also adopt a split design; a split surgical instrument may comprise a stapler and a staple cartridge. The stapler and the staple cartridge can be assembled into a whole for use according to a certain assembly relationship. In the split design, the same stapler can adapt to various staple cartridges during operation.

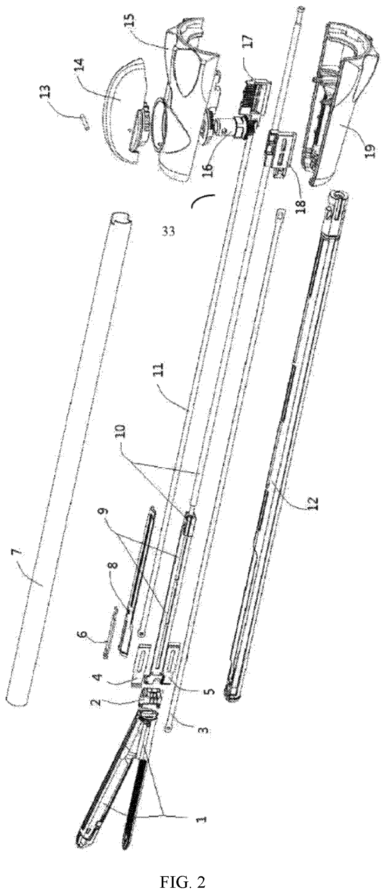

With reference to FIG. 2, the controller 30 comprises an articulation control mechanism 33. As shown in the figure, the articulation control mechanism 33 uses a gear reversing mechanism to control the articulation of the effector 1 in two directions. Specifically, the articulation control mechanism 33 comprises an articulation control gear 16 between a rotating head upper-cover 15 and a rotating head lower-cover 19, a left articulation control rack 18, and a right articulation control rack 17, wherein the rotating head upper-cover 15 and the rotating head lower-cover 19 are located at a proximal end of the extension tube 20. It can be understood that the articulation control gear 16, the left articulation control rack 18, and the right articulation control rack 17 can also be disposed in the extension tube 20.

Further, the controller 30 can include a handle component; specifically, the controller 30 comprises a rotation knob 14, a stationary handle 32, a movable handle 31 (for example, a drive trigger) relatively movably connected to the stationary handle, and a transmission mechanism for transferring an action of the movable handle 31 to the effector 1. The drive trigger 31 can be pulled to realize the functions of the effector 1, such as consecutively clamping, suturing, and cutting a human tissue. An operating region of the rotation knob 14 is located outside the rotating head upper-cover 15 or the rotating head lower-cover 19; and the operator rotates the rotation knob 14 via the operating region, so as to rotate the effector.

Preferably, when the rotation knob 14 rotates to each specific position, the effector 1 correspondingly rotates a specific angle. The effector 1 can rotate different angles, so as to adapt to different clinical applications. The two functions of rotating the rotation knob 14 to rotate the effector 1 and operating the drive trigger to drive the effector to operate are independent from each other. Generally speaking, the operating steps of the surgical instrument of the present application are as follows: first operating the rotation knob to control the effector to rotate a clinically required appropriate angle, then adjusting the position of a tissue in an effector jaw, and finally operating the drive trigger to complete the functions of clamping, suturing, and cutting the tissue.

The operating steps are described in detail hereafter by taking an integral endoscopic stapler as an example.

With reference to FIG. 2, FIG. 2 shows an exploded view of a part of the surgical instrument according to one embodiment of the present application. The proximal end of the effector 1 is connected to the distal end of the extension tube 20 via an intermediate connecting portion, wherein the intermediate connecting portion comprises a connecting assembly 2. In a preferred embodiment, the connecting assembly 2 is connected to the extension tube 20 and the effector 1 via hinges. As an example, two hinges are in parallel with each other in space, and are perpendicular to a plane in which the effector can pivot.

As an example, the extension tube 20 of the endoscopic stapler comprises an outer sleeve tube 7, an upper inner tube 8, a lower inner tube 12, a left bendable pull beam 3, and a right bendable pull beam 11. In the shown embodiment, the upper inner tube 8, the lower inner tube 12, and the outer sleeve tube 7 are coaxially arranged. The length of the lower inner tube 12 is greater than the length of the upper inner tube 8. Preferably, the length of the upper inner tube 8 is equivalent to the length of a cutter frame assembly 9. The left articulation control rack 18 and the right articulation control rack 17 are respectively disposed on the two sides of the lower inner tube 12. The left articulation control rack 18 is fixedly connected to a proximal end of the left bendable pull beam 3; and the right articulation control rack 17 is fixedly connected to a proximal end of the right bendable pull beam 11. The left bendable pull beam 3 and the right bendable pull beam 11 respectively penetrate through the connecting assembly 2, and are connected to left and right sides of the effector 1 along a central shaft. The articulation control gear 16 is disposed between the left articulation control rack 18 and the right articulation control rack 17, and is respectively engaged with the left articulation control rack 18 and the right articulation control rack 17. An upper side of the articulation control gear 16 is connected to the rotation knob 14, such that the rotation of the rotation knob 14 drives the articulation control gear 16 to rotate; then the rotation of the articulation control gear 16 drives the left articulation control rack 18 and the right articulation control rack 17 to move forward or backward (equivalent to moving towards a proximal end or a distal end), and correspondingly drives the left bendable pull beam 3 to move forward or backward; and the right articulation control rack 17 drives the right bendable pull beam 11 to move forward or backward.

With reference to FIG. 2, the rotation knob 14 is rotatably connected to the rotating head upper-cover 15 via a rotation knob pin 13; the rotating head upper-cover 15 is provided with a through hole; a part of the rotation knob 14 penetrates through the through hole, and is connected to the articulation control gear 16 between the rotating head upper-cover 15 and the rotating head lower-cover 19.

The stapler comprises a drive assembly, wherein the drive assembly comprises a slider, a cutter, a cutter frame assembly 9, and a center rod assembly 10. The cutter is used to cut a tissue between multiple rows of stapling sutures after the tissue is sutured with suturing staples.

According to one example of the present application, a part of the cutter frame assembly 9 of the drive assembly is disposed in the extension tube 20, and penetrates through the connecting assembly 2. The cutter and the slider are disposed in the effector 1.

With reference to FIG. 2, cutter bar protection plates 4 and 5 are also disposed at a connection between the cutter frame assembly 9 and the connecting assembly 2.

As shown in figures 1-3, according to one embodiment of the present application, in an initial state, the effector 1 and the central shaft of the extension tube 20 are collinear in a straight state. The left bendable pull beam 3 and the distal end of the right bendable pull beam 11 are respectively fixedly connected to the effector 1 via, for example, a rivet. In such design, connection positions between the left bendable pull beam 3, the right bendable pull beam 11 and the effector 1 deflect from a centrosymmetric plane in which the rotary shafts of the two hinges are located; and the left bendable pull beam 3 and the right bendable pull beam 11 do not pass through the centres of the hinges. Therefore, the two pull beams, by taking the hinges as rotary shafts, provide a rotation torque to the effector to drive the effector 1 to rotate.

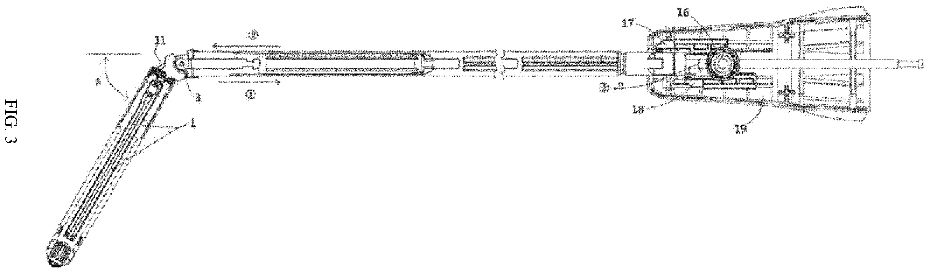

With reference to FIG. 3, FIG. 3 shows a schematic diagram when the effector of the surgical instrument rotates to a first side and a partial view of the effector according to one embodiment of the present application. The rotation knob 14 rotates anticlockwise (as shown by the arrow A), and drives the articulation control gear 16 to rotate anticlockwise; since the right articulation control rack 17 and the left articulation control rack 18 are both engaged with the articulation control gear 16, the right articulation control rack 17 is driven to move forward, and the right bendable pull beam 11 is slackened (as shown by the arrow B); simultaneously, the left articulation control rack 18 is driven to move backward, and the left bendable pull beam 3 is tightened (as shown by the arrow C); and the effector 1 rotates anticlockwise a certain angle relative to the extension tube 2. The rotation angle of the effector 1 can be controlled by controlling the rotation amount of the rotation knob 14. According to one embodiment of the present application, α can be any value between 0° and 180° (for example 0°, 30°, 45°, 60°, 90°, 120°, 135°, 180° and the like).

It can be seen from FIG. 3 that the left bendable pull beam 3 is tightened, and the right bendable pull beam 11 is slackened, so as to drive the effector to pivot. In the embodiment, the articulation control gear 16 is engaged with the right articulation control rack 17 and the left articulation control rack 18; therefore, the right articulation control rack 17 is driven to move forward, and the right bendable pull beam 11 is slackened; the two racks move in opposite directions; when the effector rotates, the tightened left bendable pull beam 3 and the slackened right bendable pull beam 11 generate equal displacements which cancel each other out, only causing rotation. In the design of the present application, the displacements of the two pull beams in opposite directions can be neutralized by other means.

The principle that the effector rotates to a second side is similar to the above principle of rotating to the first side. The differences are that the right bendable pull beam 11 is tightened, and the left bendable pull beam 3 is slackened. For example, the rotation knob 14 rotates clockwise, and drives the right bendable pull beam 11 and the left bendable pull beam 3 to move in a direction opposite to the direction as shown in FIG. 3; therefore, the effector is driven to rotate clockwise.

The transmission system of the present application has a small tolerance accumulation; the rotation angle α of the rotation knob 14 basically can be losslessly fed back to an articulation angle β of the effector, thereby having a high transmission efficiency; by adjusting the rotation angle α of the rotation knob 14, the articulation angle β of the effector can be controlled; theoretically, α can be any value between 0° and 180°, for example, 0°, 30°, 45°, 60°, 90°, 120°, 135°, 180° and the like; and β can be any value between 0° and 180°, for example 0°, 30°, 45°, 60°, 90°, 120°, 135°, 180° and the like.

In a specific embodiment, the angle relationship between α and β satisfies the following formula:

$$\beta = 0.9\,\alpha - 2.1$$

In another embodiment, the left bendable pull beam 3 and the right bendable pull beam 11 can be divided into two segments or multiple segments. If the bendable pull beams are segmented, detachment can be achieved.

In another embodiment, two or more bendable pull beams can be placed in an overlapping manner.

In another embodiment, the rotation knob 14 does not consecutively rotate; instead, a plurality of fixed rotation gears are configured, and the gears have a 2°, 5°, or 10° difference therebetween.

The surgical instrument of the present application can be applied to lung and digestive tract (including stomach and intestine) operations. A lung operation is generally to cut off a nidus. A digestive tract operation generally needs to join the cut intestine after the nidus is cut off.

Generally, the effector of a pivotable endoscopic stapler can articulate; the proximal end of the effector is connected to the articulation control mechanism (for example, the articulation control gear 16 in the figure) inside the controller (for example, a stapler handle); and the effector can be controlled to rotate to multiple angles by adjusting the articulation control mechanism. In the initial state, the effector and the central shaft of the extension tube are collinear in a straight state; the effector of the stapler extends into a thoracic cavity or an abdominal cavity via a puncture; the effector is controlled to rotate a certain angle by controlling the articulation control mechanism on the handle, so as to complete a series of operations such as clamping, transversely cutting, and stapling a surgical site. After the operation is completed, the effector is controlled to become straight again and back out of the body by controlling the articulation control mechanism on the handle.

For instance, for a digestive tract operation, in clinic, in order to completely remove a tumor and prevent tumor tissue residue, the position of the transverse cutting line is usually about 5 cm away from the tumor boundary. When the position of a rectal cancer tumor is close to a low position of the anus of the patient, the effector of the stapler needs to be placed at a lower position in the narrow pelvic cavity. Affected by the hip bone, the entrance position of the stapler is limited, and a stapler having an effector with a small articulation angle cannot cut off the rectum perpendicularly, thereby increasing the surgical risk of incomplete tumor removal.

A surgical instrument having an effector with a small articulation angle cannot cut off the rectum perpendicularly, while a stapler having an effector with a large articulation angle can. Furthermore, a surgical instrument having an effector with a large articulation angle can cope with a tumor at a lower position. When the rotation angle of the surgical instrument is larger, the tumor can be cut off at a lower position; and the surgical instrument can be better applied to a low rectal cancer operation or other in-vivo space-limited situations.

In the surgical instrument according to the present application, the articulation angle of the effector can reach 90° and even 180°, so as to adapt to extreme application situations.

According to another embodiment of the present application, as a substitution, the left bendable pull beam 3 and the right bendable pull beam 11 can also be a wire, for example, a flexible component wound with single strand of wire or multiple strands of wires. It should be understood that the bendable pull beams and the wire are provided as an example but not a limitation, and a person skilled in the art can construct more other similar flexible components to implement the articulation of the effector. However, such an implementation mode should not be interpreted to depart from the scope of the present application.

In addition, it should also be understood that the embodiments described above can also be implemented in an integrally designed surgical instrument such as an endoscopic stapler and the like. In addition, the various embodiments can also be implemented in other products such as a closer, an electrotome and the like.

The surgical instrument of the present application adopts the flexible components to pivot the effector relative to the distal end of the extension tube, such that the effector can pivot a larger angle, thereby being applicable to medical treatment under a more complicated clinical condition, for example, the surgical instrument can more simply implement an operation in a limited space (such as thoracic cavity and pelvic cavity). Therefore, the present application solves the problem that other surgical instruments cannot solve. The effector of the present application can pivot a large angle; and the surgical instrument can complete the functions of nidus cutting, tissue cutting and suturing at difficult locations, thereby benefiting more patients.

The above descriptions and illustrations are provided only as descriptive examples. Any citation to a claim element in a singular form, for example the citation using the article "a/an", "certain", or "the", should not be considered to limit the element to be singular. For each specific application, a person skilled in the art can use different methods to implement the described structure. However, such implementation mode should not be interpreted to depart from the scope of the present application.

The earlier descriptions of the disclosed embodiments are provided for the purpose of enabling a person skilled in the art to make or use the present application. Various modifications to the embodiments would be obvious for a person skilled in the art; and the general principles defined herein can be applied to other embodiments without departing from the spirit or scope of the present application. Therefore, the present application is not intended to limit the embodiments shown herein, but should be granted with the broadest sense scope consistent with the principles and novel features disclosed herein and in the accompanying claims.

**Claims**

1. A surgical instrument with a pivotable effector, **characterized in that** the surgical instrument comprises an effector at a distal end, a controller at a proximal end, and an extension tube between the effector and the controller, wherein the extension tube is movably connected to a distal end of the controller; a distal end of the extension tube is pivotally connected to a proximal end of the effector; the extension tube forms a connection channel for transferring an action of the controller to the effector; the controller comprises an articulation control mechanism capable of moving in at least one direction; the extension tube comprises a first flexible component, and moves in at least one direction through the articulation control mechanism, and the first flexible component provides a rotation torque for the effector, and drives the effector to rotate towards at least one side relative to the distal end of the extension tube.

2. The surgical instrument with a pivotable effector according to claim 1, **characterized in that** the distal end of the extension tube is pivotally connected to the proximal end of the effector via a hinge; the first flexible component does not pass through a centre of the hinge, such that the first flexible component takes the hinge as a rotation axis, provides a rotation torque for the effector, and drives the effector to rotate to at least one side relative to the distal end of the extension tube.

3. The surgical instrument with a pivotable effector according to claim 2, **characterized in that** the effector rotates in a plane perpendicular to the hinge rotation axis.

4. The surgical instrument with a pivotable effector according to any one of claims 1-3, **characterized in that** the articulation control mechanism comprises an operating component, a articulation control gear, and a first articulation control rack, wherein the operating component is connected to the articulation control gear, and drives the articulation control gear to rotate; the articulation control gear is engaged with the first articulation control rack; the rotation of the articulation control gear drives the first articulation control rack to move forward or backward; and the first articulation control rack is fixedly connected to a proximal end of the first flexible component.

5. The surgical instrument with a pivotable effector according to any one of claims 1-3, **characterized in that** the extension tube further comprises a second flexible component; a distal end of the first flexible component and a distal end of the second flexible component are respectively connected on the two sides of the effector along a central shaft; when the articulation control mechanism moves in a first direction, the articulation control mechanism drives the first flexible component to move towards a proximal end thereof, drives the second flexible component to move towards a distal end thereof, and then drives the effector to rotate to a first side relative to the distal end of the extension tube; and when the articulation control mechanism moves in a second direction, the articulation control mechanism drives the first flexible component to move towards the distal end thereof, drives the second flexible component to move towards the proximal end thereof, and then drives the effector to rotate to a second side relative to the distal end of the extension tube.

6. The surgical instrument with a pivotable effector according to claim 4, **characterized in that** the extension tube further comprises a second flexible component; a distal end of the first flexible component and a distal end of the second flexible component are respectively connected on the two sides of the effector along a central shaft; when the articulation control mechanism moves in a first direction, the articulation control mechanism drives the first flexible component to move towards a proximal end thereof, drives the second flexible component to move towards a distal end thereof, and then drives the effector to rotate to a first side relative to the distal end of the extension tube; and when the articulation control mechanism moves in a second direction, the articulation control mechanism drives the first flexible component to move towards the distal end thereof, drives the second flexible component to move towards the proximal end thereof, and then drives the effector to rotate to a second side relative to the distal end of the extension tube; the articulation control mechanism further comprises a second articulation control rack; the articulation control gear is engaged with the second articulation control rack; the rotation of the articulation control gear drives the second articulation control rack to move forward or backward; and the second articulation control rack is fixedly connected to a proximal end of the second flexible component.

7. The surgical instrument with a pivotable effector according to claim 5 or 6, **characterized in that** the first flexible component and the second flexible component are segmented flexible components.

8. The surgical instrument with a pivotable effector according to claim 6, **characterized in that** the operating component is a rotation knob, and an operating region of the rotation knob is located outside the extension tube.

9. The surgical instrument with a pivotable effector according to claim 8, **characterized in that** the extension tube comprises an outer sleeve tube, an upper inner tube, and a lower inner tube, the upper inner tube, the lower inner tube, and the outer sleeve tube being coaxially arranged.

10. The surgical instrument with a pivotable effector according to claim 5, **characterized in that** the first flexible component and the second flexible component are a bendable pull beam or a wire rope.

11. The surgical instrument with a pivotable effector according to claim 6, **characterized in that** the first flexible component and the second flexible component are a bendable pull beam or a wire rope.

12. The surgical instrument with a pivotable effector according to claim 9, **characterized in that** the first flexible component is a left bendable pull beam; the second flexible component is a right bendable pull beam; the first articulation control rack is a left articulation control rack; the second articulation control rack is a right articulation control rack; the left articulation control rack and the right articulation control rack are respectively disposed on the two sides of the lower inner tube; the left bendable pull beam and the right bendable pull beam respectively penetrate through a connecting assembly, and are connected to left and right sides of the effector along the central shaft; an upper side of the articulation control gear is connected to the rotation knob, such that the rotation of the rotation knob drives the articulation control gear to rotate, then drives the left articulation control rack and the right articulation control rack to move forward or backward, and correspondingly drives the left bendable pull beam to move forward or backward; and the right articulation control rack drives the right bendable pull beam to move forward or backward.

13. The surgical instrument with a pivotable effector according to any one of claims 1-3, **characterized in that** a rotating head upper-cover and a rotating lower-cover which are connected to each other are disposed at a proximal end of the extension tube; the articulation control mechanism is located between the rotating head upper-cover and the rotating lower-cover; and the rotation knob is rotatably connected to the rotating head upper-cover via a rotation knob pin.

14. The surgical instrument with a pivotable effector according to any one of claims 1-3, 6, and 8-12, **characterized in that** a rotation angle of the effector to at least one side relative to the distal end of the extension tube is any value between 0° and 180°.

15. The surgical instrument with a pivotable effector according to any one of claims 1-3, 6, and 8-12, **char-**

**acterized in that** the surgical instrument is an endoscopic stapler.

FIG. 1

FIG. 2

FIG. 3

## INTERNATIONAL SEARCH REPORT

| | International application No. |
|---|---|
| | **PCT/CN2019/114934** |

**A. CLASSIFICATION OF SUBJECT MATTER**

A61B 17/072(2006.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNABS, CNTXT: 执行器, 弯, 转, 齿轮, 齿条, 柔, 软, 弹, 拉, 牵; VEN, USTXT, WOTXT, EPTXT: effector, bend, curv+, rotat +, gear, rack, soft, pliable, flexibl+, elast+, pull, stretch

**C. DOCUMENTS CONSIDERED TO BE RELEVANT**

| Category* | Citation of document, with indication, where appropriate, of the relevant passages | Relevant to claim No. |
|---|---|---|
| X | CN 105434002 A (EZISURG (SUZHOU) MEDICAL TECHNOLOGY CO., LTD. et al.) 30 March 2016 (2016-03-30) description, paragraphs 7-67, and figures 1-9 | 1-12, 14, 15 |
| Y | CN 105434002 A (EZISURG (SUZHOU) MEDICAL TECHNOLOGY CO., LTD. et al.) 30 March 2016 (2016-03-30) description, paragraphs 7-67, and figures 1-9 | 13 |
| Y | CN 104546048 A (SHANGHAI EZISURG MEDICAL CO., LTD. et al.) 29 April 2015 (2015-04-29) description, paragraphs 87-122, and figures 1-14 | 13 |
| X | CN 205339036 U (EZISURG (SUZHOU) MEDICAL TECHNOLOGY CO., LTD. et al.) 29 June 2016 (2016-06-29) description, paragraphs 7-67, and figures 1-9 | 1-12, 14, 15 |
| X | US 5405344 A (ETHICON, INC.) 11 April 1995 (1995-04-11) description, column 12, line 50 to column 13, line 15, and figures 21 and 22 | 1-3, 5, 7, 10-12, 14, 15 |
| A | CN 2751749 Y (WENG, Zhiqiang) 18 January 2006 (2006-01-18) entire document | 1-15 |

☐ Further documents are listed in the continuation of Box C.　　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **29 November 2019** | **07 January 2020** |

| Name and mailing address of the ISA/CN | Authorized officer |
|---|---|
| **China National Intellectual Property Administration (ISA/CN)** **No. 6, Xitucheng Road, Jimenqiao Haidian District, Beijing 100088 China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2019/114934**

| Patent document cited in search report | | | Publication date (day/month/year) | Patent family member(s) | | | Publication date (day/month/year) |
|---|---|---|---|---|---|---|---|
| CN | 105434002 | A | 30 March 2016 | WO | 2017063382 | A1 | 20 April 2017 |
| | | | | JP | 2018532530 | A | 08 November 2018 |
| | | | | BR | 112018007509 | A2 | 18 December 2018 |
| | | | | US | 2018289372 | A1 | 11 October 2018 |
| | | | | EP | 3363372 | A1 | 22 August 2018 |
| | | | | CN | 105434002 | B | 17 November 2017 |
| CN | 104546048 | A | 29 April 2015 | ES | 2720593 | T3 | 23 July 2019 |
| | | | | EP | 3222221 | B1 | 13 February 2019 |
| | | | | JP | 6403902 | B2 | 10 October 2018 |
| | | | | WO | 2016115917 | A1 | 28 July 2016 |
| | | | | US | 10342537 | B2 | 09 July 2019 |
| | | | | EP | 3222221 | A1 | 27 September 2017 |
| | | | | BR | 112017013651 | A2 | 06 March 2018 |
| | | | | JP | 2018501928 | A | 25 January 2018 |
| | | | | CN | 104546048 | B | 16 November 2016 |
| | | | | US | 2017340327 | A1 | 30 November 2017 |
| CN | 205339036 | U | 29 June 2016 | None | | | |
| US | 5405344 | A | 11 April 1995 | CA | 2133182 | A1 | 31 March 1995 |
| | | | | EP | 0646356 | A2 | 05 April 1995 |
| | | | | EP | 0646356 | A3 | 12 July 1995 |
| | | | | JP | H07163574 | A | 27 June 1995 |
| | | | | AU | 7431694 | A | 13 April 1995 |
| | | | | AU | 682710 | B2 | 16 October 1997 |
| CN | 2751749 | Y | 18 January 2006 | None | | | |

Form PCT/ISA/210 (patent family annex) (January 2015)

**EP 3 875 041 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20100237131 A1
- CN 201510672007
- CN 201510026576X